# FASCICULE DE BREVET EUROPEEN

(11) **EP 0 546 887 B1**
(45) Date de publication et mention de la délivrance du brevet: **26.06.1996**
(21) Numéro de dépôt: 92403224.6
(22) Date de dépôt: 30.11.1992
(51) Int. Cl.: G02B 23/14, A61B 3/113

(54) **Dispositif pour déterminer la portion d'un champ regardée par l'oeil d'un observateur**
Anordnung zur Bestimmung desjenigen Teils eines Bildfeldes, das vom Auge eines Beobachters betrachtet wird
Device for determining the portion of a field of view observed by the eye of a spectator

(30) Priorité: 13.12.1991 FR 9115509
(43) Date de publication de la demande: 16.06.1993
(73) Titulaire: AEROSPATIALE Société Nationale Industrielle, F-75781 Paris Cédex 16 (FR)
(72) Inventeur: Acier, Bruno, F-28000 Chartres (FR); Merle, Jean-Pierre, F-91400 Orsay (FR); Roze des Ordons, Jacques, F-91600 Savigny sur Orge (FR)
(74) Mandataire: Bonnetat, Christian

(56) Documents cités:
- EP-A- 0 434 289
- FR-A- 2 522 804
- FR-A- 2 561 371
- US-A- 4 852 988
- PATENT ABSTRACTS OF JAPAN vol. 15, no. 305 (P-1234), 5 août 1991; & JP-A-3 107 934

## Description

La présente invention concerne un dispositif pour déterminer la portion d'un champ regardée par l'oeil d'un observateur, un tel dispositif étant généralement appelé "oculomètre" dans la technique.

On connaît déjà, par exemple par le brevet EP-A-0 157 973, un dispositif destiné à déterminer la portion d'un champ regardée par l'oeil d'un observateur et comprenant :
- une source de lumière adressant un faisceau lumineux sur la cornée dudit oeil pour former un reflet cornéen ;
- des premiers moyens de prise de vue observant ledit reflet cornéen et engendrant des premiers signaux électriques représentatifs de l'orientation de l'axe optique dudit oeil ;
- des deuxièmes moyens de prise de vue observant ledit champ et engendrant des deuxièmes signaux électriques représentatifs dudit champ ; et
- des moyens de calcul recevant lesdits premiers et deuxièmes signaux électriques et délivrant des troisièmes signaux électriques représentatifs de la portion dudit champ regardée par ledit oeil.

Le fonctionnement d'un tel dispositif est basé notamment sur les enseignements contenus dans la publication de MACKWORTH et al, "Eye Fixations Recorded on Changing Visual Scenes by the Television Eye Marker" dans Journal of the Optical Society of America, juillet 1958, et montrant qu'il y a corrélation entre la variation en orientation de l'axe optique de l'oeil et le déplacement dudit reflet cornéen.

Par suite, en observant ledit reflet cornéen, lesdits premiers moyens de prise de vue connaissent, à chaque instant, la position du reflet cornéen et l'orientation de l'axe optique de l'oeil. Lesdits moyens de calcul peuvent donc déterminer la portion du champ regardée, à chaque instant, par ledit oeil, puisqu'ils reçoivent les informations de champ provenant des deuxièmes moyens de prise de vue.

Lesdits premiers et deuxièmes moyens de prise de vue peuvent être du type caméra, par exemple du type CCD et, de préférence, ladite source de lumière est invisible (infrarouge) pour ne pas gêner l'observateur. Elle est généralement du type diode LED.

Selon une variante de mise en oeuvre connue, on peut associer, audit reflet cornéen, le reflet rétinien engendré par ledit faisceau lumineux et diaphragmé par la pupille, afin de déterminer l'orientation de l'axe optique de l'oeil.

Dans les dispositifs connus, du type décrit ci-dessus, l'oeil de l'observateur regarde directement le champ, sans interposition d'un système optique, tel qu'une lunette de visée, limitant la vision périphérique. Il n'y a donc aucun obstacle à l'illumination de l'oeil par la source de lumière invisible.

En revanche, si l'on désire prévoir un système optique pour l'observation du champ par l'oeil de l'observateur, on se heurte à des difficultés.

En effet, l'oeil doit se trouver proche de l'oculaire du système optique, dans le plan de la pupille dudit système optique. Par suite, il n'est pas possible, faute de place, d'introduire le faisceau lumineux invisible entre l'oculaire du système optique et l'oeil. Par ailleurs, si l'on pensait illuminer l'oeil en lumière invisible à travers ledit système optique, le faisceau de lumière invisible serait limité en diamètre par la pupille dudit système optique. Etant donné que la surface de la pupille d'un système optique est petite par rapport à la surface de la cornée à illuminer, il en résulterait une impossibilité d'illuminer la totalité de la cornée et donc de mesurer toutes les orientations de l'axe optique de l'oeil.

La présente invention a pour objet de remédier à ces inconvénients et de permettre de doter d'un système optique les dispositifs du type décrit ci-dessus.

A cette fin, selon l'invention, le dispositif destiné à déterminer la portion d'un champ regardée par l'oeil d'un observateur et comprenant :
- une source de lumière adressant un faisceau lumineux sur ledit oeil pour former au moins un reflet ;
- des premiers moyens de prise de vue observant ledit reflet et engendrant des premiers signaux électriques représentatifs de l'orientation de l'axe optique dudit oeil ;
- des deuxièmes moyens de prise de vue observant ledit champ et engendrant des deuxièmes signaux électriques représentatifs dudit champ ; et
- des moyens de calcul recevant lesdits premiers et deuxièmes signaux électriques et délivrant des troisièmes signaux électriques représentatifs de la portion dudit champ regardée par ledit oeil ;
   est remarquable en ce qu'il comprend :
- un système optique de visée à travers lequel ledit oeil regarde ledit champ, ledit système optique comprenant un oculaire de sortie derrière lequel est disposé ledit oeil ; et
- un premier élément optique disposé du côté du plan focal objet dudit oculaire de sortie, ledit premier élément optique laissant passer les rayons lumineux qui proviennent dudit champ et qui traversent ledit système optique et adressant audit oeil ledit faisceau lumineux émis par ladite source de lumière.

Ainsi, ledit faisceau lumineux, destiné à illuminer l'oeil, passe à travers ledit oculaire de sortie. Ce dernier a donc un double rôle, à savoir i) adresser l'image du champ à l'oeil à travers la pupille de sortie du système optique de visée et ii) illuminer ledit oeil à l'aide de la lumière émise par ladite source, sans limitation par ladite pupille de sortie.

De préférence, notamment lorsque le dispositif est destiné à déterminer la portion de champ regardée par l'oeil par la méthode connue mettant en oeuvre, outre ledit reflet cornéen, le reflet rétinien engendré par ledit faisceau lumineux (méthode dite du "vecteur cornéen"), ledit premier élément optique est disposé dans le plan focal objet dudit oculaire de sortie.

Selon un premier mode de réalisation, ledit premier élément optique, qui peut être du type lame parallèle à surfaces traitées, est, d'un côté, au moins partiellement transparent pour les rayons lumineux provenant dudit champ et, de l'autre côté, partiellement transparent et partiellement réfléchissant pour ledit faisceau lumineux émis par ladite source de lumière.

Par suite, la partie dudit faisceau lumineux réfléchie par l'oeil peut traverser ledit premier élément optique et être dirigée vers un second élément optique, couplé optiquement avec lesdits premiers et deuxièmes moyens de prise de vue et disposé en amont (par rapport au sens de propagation des rayons lumineux provenant dudit champ) dudit premier élément optique, ledit second élément optique étant, d'un côté, partiellement transparent et partiellement réfléchissant pour les rayons lumineux provenant dudit champ et, de l'autre côté, au moins partiellement réfléchissant pour ledit faisceau lumineux émis par ladite source de lumière.

Dans ce cas, il est avantageux que ledit système optique de visée comporte un véhicule optique afocal définissant un point de convergence intermédiaire, à l'emplacement duquel est disposé ledit second élément optique.

Le système optique de visée peut alors comporter une lunette de visée, du type lunette astronomique, de laquelle est solidarisé ledit véhicule optique, celui-ci comportant un objectif couplé optiquement à l'oculaire de ladite lunette de visée et un oculaire formant ledit oculaire de sortie dudit système optique.

On remarquera que ce mode de réalisation est particulièrement avantageux, car il permet d'adapter ladite lunette de visée, sans modification de celle-ci, pour l'utiliser dans le dispositif conforme à la présente invention. On obtient ainsi un capteur destiné à être couplé à une lunette de visée usuelle pour la rendre apte au dispositif de l'invention.

Aussi, la présente invention concerne également un capteur destiné à former avec une lunette de visée un système optique de visée pour un dispositif destiné à déterminer la portion d'un champ regardée par l'oeil d'un observateur, ledit capteur comprenant :
- une source de lumière adressant un faisceau lumineux sur ledit oeil pour former au moins un reflet ;
- des premiers moyens de prise de vue observant ledit reflet et engendrant des premiers signaux électriques représentatifs de l'orientation de l'axe optique dudit oeil ;
- des deuxièmes moyens de prise de vue observant ledit champ et engendrant des deuxièmes signaux électriques représentatifs dudit champ ; et
- des moyens de calcul recevant lesdits premiers et deuxièmes signaux électriques et délivrant des troisièmes signaux électriques représentatifs de la portion dudit champ regardée par ledit oeil.

Selon la présente invention, un tel capteur est remarquable en ce qu'il comporte :
- un véhicule optique afocal solidarisé de ladite lunette de visée, ledit véhicule optique comportant un objectif couplé à l'oculaire de ladite lunette de visée et un oculaire formant l'oculaire de sortie dudit système optique ;
- un premier élément optique disposé du côté du plan focal objet dudit oculaire de sortie et adressant audit oeil ledit faisceau lumineux émis par ladite source de lumière, ledit premier élément optique étant, d'un côté, au moins partiellement transparent pour les rayons lumineux provenant dudit champ et, de l'autre côté, partiellement transparent et partiellement réfléchissant pour ledit faisceau lumineux émis par ladite source de lumière ; et
- un second élément optique couplé optiquement avec lesdits premiers et deuxièmes moyens de prise de vue et disposé entre ledit objectif et ledit premier élément optique, ledit second élément optique étant, d'un côté, partiellement transparent et partiellement réfléchissant pour les rayons lumineux provenant dudit champ et, de l'autre côté, au moins partiellement réfléchissant pour ledit faisceau lumineux émis par ladite source de lumière.

De préférence, afin de former une entité de construction, ladite source de lumière et/ou lesdits premiers et deuxièmes moyens de prise de vue sont incorporés dans ledit capteur.

Selon un deuxième mode de réalisation, ledit premier élément optique, qui peut également être du type lame parallèle à surfaces traitées, est, d'un côté, partiellement transparent et partiellement réfléchissant pour les rayons lumineux provenant dudit champ et, de l'autre côté, au moins partiellement réfléchissant pour ledit faisceau lumineux émis par ladite source de lumière.

Ainsi, ledit premier élément optique peut être couplé optiquement avec lesdits premiers et deuxièmes moyens de prise de vue, le couplage avec lesdits premiers moyens de prise de vue étant obtenu grâce à un troisième élément optique, d'un côté au moins partiellement transparent et de l'autre côté au moins partiellement réfléchissant pour ledit faisceau lumineux émis par ladite source de lumière et permettant de séparer de ce faisceau la partie qui est réfléchie par l'oeil et par ledit premier élément optique.

Dans ce cas, il est avantageux que lesdits premier et troisième éléments optiques soient incorporés à une lunette de visée pour former ledit système optique, l'oculaire de ladite lunette de visée formant l'oculaire de sortie dudit système optique.

On obtient alors, par modification d'une lunette de visée usuelle, un système optique tout spécialement destiné au dispositif conforme à la présente invention.

Ainsi, la présente invention concerne également un système optique de visée pour un dispositif destiné à déterminer la portion d'un champ regardée par l'oeil d'un observateur, ledit système comprenant :
- une source de lumière adressant un faisceau lumineux sur ledit oeil pour former au moins un reflet ;
- des premiers moyens de prise de vue observant ledit reflet et engendrant des premiers signaux électriques représentatifs de l'orientation de l'axe optique dudit oeil ;
- des deuxièmes moyens de prise de vue observant ledit champ et engendrant des deuxièmes signaux électriques représentatifs dudit champ ; et
- des moyens de calcul recevant lesdits premiers et deuxièmes signaux électriques et délivrant des troisièmes signaux électriques, représentatifs de la portion dudit champ regardée par ledit oeil ;
ledit système optique étant remarquable en ce qu'il comporte :
- une lunette de visée pourvue d'un oculaire ;
- un premier élément optique disposé du côté du plan focal objet dudit oculaire de la lunette de visée et adressant audit oeil ledit faisceau lumineux émis par ladite source de lumière, ledit premier élément optique étant, d'un côté, partiellement transparent et partiellement réfléchissant pour les rayons lumineux provenant dudit champ et, de l'autre côté, au moins partiellement réfléchissant pour ledit faisceau lumineux émis par ladite source de lumière, ledit premier élément optique étant optiquement couplé auxdits deuxièmes moyens de prise de vue ; et
- un troisième élément optique, d'un côté au moins partiellement transparent et de l'autre côté au moins partiellement réfléchissant pour ledit faisceau lumineux émis par ladite source de lumière, ledit troisième élément optique assurant la séparation entre ledit faisceau lumineux et la partie de celui-ci réfléchie par l'oeil et ledit premier élément optique, ainsi que le couplage optique entre ledit premier élément optique et lesdits premiers moyens de prise de vue.

De préférence, afin de former un ensemble unitaire, ladite source de lumière et/ou lesdits premiers et deuxièmes moyens de prise de vue sont incorporés dans ledit système optique.

Les figures du dessin annexé feront bien comprendre comment l'invention peut être réalisée. Sur ces figures, des références identiques désignent des éléments semblables.

La figure 1 montre schématiquement un premier mode de réalisation du dispositif conforme à la présente invention.

La figure 2 montre schématiquement l'image d'un champ vu à travers le dispositif de l'invention.

La figure 3 est une vue partielle, agrandie et en coupe, du dispositif de la figure 1.

La figure 4 illustre en coupe partielle un second mode de réalisation du dispositif conforme à la présente invention.

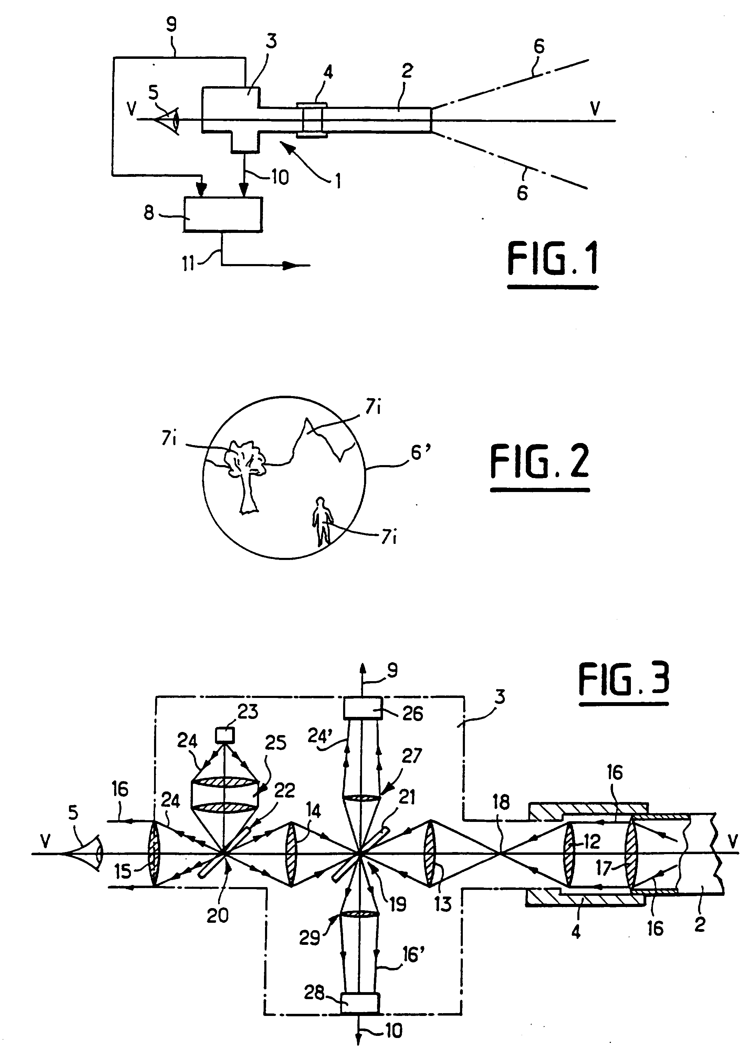

Le dispositif 1, conforme à la présente invention et représenté schématiquement sur la figure 1, comporte une lunette de visée 2, par exemple du type lunette astronomique, et un capteur optique-électrique 3, solidarisés l'un de l'autre par un élément de fixation 4, de façon que leurs axes optiques soient rigoureusement alignés sur un axe optique commun définissant une ligne de visée V-V.

Un oeil 5 d'un observateur est disposé sur la ligne de visée V-V, derrière le capteur 3, de sorte qu'il peut regarder le champ 6 défini par la lunette de visée 2. Comme l'illustre la figure 2, l'oeil 5 voit donc l'image 6' du champ 6 à travers la lunette 2 et le capteur 3, ainsi que les images des différents éléments ou portions 7i composant ce champ.

Par ailleurs, le dispositif 1 de la figure 1 comporte un calculateur 8 recevant des signaux électriques de sorties 9 et 10 du capteur 3 et les traitant pour délivrer des signaux à sa sortie 11.

Comme le montre schématiquement la figure 3 sur laquelle la lunette 2 n'est représentée que partiellement, le capteur optique-électrique 3 comporte une suite de quatre lentilles convergentes 12, 13, 14 et 15, centrées sur la ligne de visée V-V et disposées le long de celle-ci de façon à former un véhicule optique afocal et achromatique.

La lentille 12 fait office d'objectif pour le capteur 3 et elle reçoit de l'oculaire 17 de la lunette de visée 2 le faisceau 16, traversant ladite lunette de visée 2 et constitué alors de rayons parallèles du fait de son passage à travers ledit oculaire 17. Elle le fait converger à son foyer image 18. Chacune des lentilles 13 et 14 fait de nouveau converger le faisceau 16, respectivement aux points intermédiaires 19 et 20. Le point 20 est confondu avec le foyer objet de la lentille 15. Ainsi, la lentille 15 fait office d'oculaire de sortie pour l'ensemble de la lunette de visée 2 et du capteur 3 et adresse à l'oeil 5 le faisceau 16, de nouveau sous forme de rayons parallèles. L'oeil 5 voit donc l'image 6' après inversion par la lentille 12, redressement par la lentille 13, inversion par la lentille 14 et redressement par l'oculaire de sortie 15.

Au point de convergence 19, entre les lentilles 13 et 14, est disposée une lame inclinée 21, qui, d'un côté, est semi-transparente et semi-réfléchissante pour le faisceau 16 et, de l'autre, au moins partiellement réfléchissante pour la lumière infrarouge.

De plus, au foyer objet 20 de l'oculaire de sortie 15, est disposée une lame inclinée 22, qui, d'un côté, est transparente pour le faisceau 16 et, de l'autre, semi-réfléchissante et semi-transparente pour la lumière infrarouge.

Par ailleurs, le capteur 3 comporte :
- une source de lumière infrarouge 23, par exemple une diode LED émettant un faisceau infrarouge 24, qui, par l'action d'une optique 25, est convergé sur la lame inclinée 22 ;
- une caméra CCD infrarouge 26, disposée en regard de la lame inclinée 21, avec interposition d'une optique 27, la sortie de la caméra 26 formant la sortie 9 du capteur 3 ; et
- une caméra CCD 28, disposée en regard de la lame inclinée 21, avec interposition d'une optique 29, la sortie de la caméra 28 formant la sortie 10 du capteur 3.

Ainsi, le faisceau 16 provenant de l'oculaire 17 de la lunette de visée 2 est adressé à l'oeil 5 par les lentilles 12 à 15 de la façon indiquée ci-dessus, en traversant les lames inclinées 21 et 22. De plus, une partie 16' du faisceau 16 est adressée à la caméra 28 par réflexion sur la lame inclinée 21 et à travers l'optique 29. Les signaux électriques apparaissant à la sortie 10 représentent donc l'image 6' du champ 6.

Quant à lui, le faisceau 24 est adressé à l'oeil 5 sous la forme d'un faisceau à rayons parallèles, grâce à la réflexion sur la lame 22 et à l'action de l'oculaire de sortie 15. Ce faisceau 24 engendre donc, par illumination de la cornée et de la rétine de l'oeil 5 et par réflexion sur celles-ci, un reflet cornéen et un reflet rétinien véhiculés par un faisceau 24', qui traverse, en sens inverse du faisceau 16, l'oculaire de sortie 15, la lame inclinée 20 et la lentille 14, pour se réfléchir sur la lame inclinée 21, avant d'être adressé à la caméra 26 par l'optique 27.

Puisque la différence de position entre ce reflet cornéen et le centre de la pupille brillante formée par le reflet rétinien est représentative de l'orientation de l'axe optique de l'oeil 5, l'information fournie à la sortie 9 de la caméra 26 est représentative de celui des éléments 7i de l'image 6', regardé par ledit oeil 5.

Par suite, le calculateur 8 recevant à ses entrées les signaux électriques provenant des sorties 9 et 10 des caméras 26 et 28 est capable de délivrer à sa sortie 11, par un traitement connu desdits signaux électriques d'entrée, des signaux électriques de sortie représentatifs dudit élément 7i regardé par l'oeil 5.

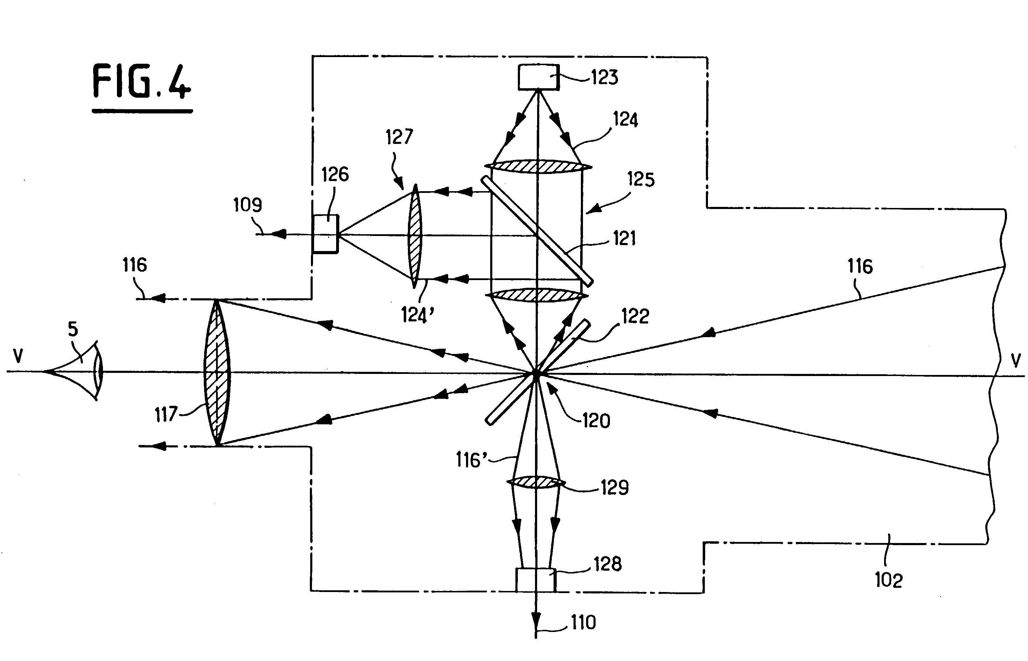

Sur la figure 4, on a représenté partiellement une lunette de visée 102, perfectionnée selon l'invention et destinée à remplacer, dans le dispositif 1 de la figure 1, l'ensemble de la lunette de visée usuelle 2 et du capteur 3. L'axe optique, ou ligne de visée, de la lunette 102 porte également la référence V-V.

La lunette 102 comporte un oculaire 117 et l'oeil 5 est disposé derrière cet oculaire pour recevoir le faisceau optique 116 traversant ladite lunette (en provenance de l'objectif non représenté de ladite lunette 102) et pour voir l'image 6 (figure 2) du champ 6. La lunette 102 comporte des sorties 109 et 110, respectivement analogues aux sorties 9 et 10, décrites ci-dessus.

Au foyer objet 120 de l'oculaire 117 est disposée une lame inclinée 122, qui, d'un côté, est partiellement transparente et partiellement réfléchissante pour le faisceau 116 et, de l'autre côté, au moins partiellement réfléchissante pour la lumière infrarouge.

Par ailleurs, la lunette 102 comporte :
- une source de lumière infrarouge 123 (diode LED), émettant un faisceau infrarouge 124, qui, par l'action d'une optique 125, est convergé sur la lame inclinée 122 ;
- une lame inclinée 121, disposée sur le trajet du faisceau 124, ladite lame 121 étant, d'un côté, au moins partiellement transparente et, de l'autre, au moins partiellement réfléchissante pour la lumière infrarouge ;
- une caméra CCD infrarouge 126, disposée en regard de la lame inclinée 121, avec interposition d'une optique 127, la sortie de la caméra 126 formant la sortie 109 de la lunette 102 ; et
- une caméra CCD 128, disposée en regard de la lame inclinée 122, avec interposition d'une optique 129, la sortie de la caméra 128 formant la sortie 110 de la lunette 102.

Ainsi, le faisceau 116 traversant la lunette de visée 102 est adressé à l'oeil 5 à travers la lame inclinée 122 et l'oculaire 117. De plus, une partie 116' du faisceau 116 est adressée à la caméra 128 par réflexion sur la lame inclinée 122 et à travers l'optique 129. Les signaux électriques apparaissent à la sortie 110 représentant donc l'image 6' du champ 6.

Le faisceau infrarouge 124 est adressé à l'oeil 5 sous la forme d'un faisceau parallèle, après traversée de la lame inclinée 121 et de l'optique 125, grâce à la réflexion sur la lame inclinée 122 et à l'action de l'oculaire 117 de la lunette 102. Ce faisceau 124 engendre donc, par illumination de la cornée et de la rétine de l'oeil 5 et par réflexion sur celles-ci, un reflet cornéen et un reflet rétinien véhiculés par un faisceau 124', qui traverse, en sens inverse du faisceau 116, l'oculaire 117 et se réfléchit sur les lames inclinées 122 et 121, avant d'être adressé à la caméra 126 par l'optique 127.

On voit donc que, sur les sorties 109 et 110, apparaissent des signaux électriques semblables respectivement à ceux apparaissant sur les sorties 9 et 10 mentionnées ci-dessus. Par suite, le calculateur 8 peut traiter de façon identique les signaux desdites sorties 109 et 110, pour délivrer à sa sortie 11 des signaux électriques représentatifs de l'élément 7i regardé par l'oeil 5 à travers la lunette 102.

De la description ci-dessus, on remarquera qu'il est aisé de réaliser, grâce à l'invention, un dispositif binoculaire en associant un système optique 2,3 ou 102 à chacun des deux yeux 5 d'un observateur. Dans ce cas, il est avantageux que le calculateur 8 traite les signaux provenant des deux systèmes optiques 2,3 ou 102.

## Revendications

1. Dispositif (1) destiné à déterminer la portion (7i) d'un champ (6) regardée par l'oeil (5) d'un observateur et comprenant :
- une source de lumière (23 ; 123) adressant un faisceau lumineux (24 ; 124) sur ledit oeil (5) pour former au moins un reflet ;
- des premiers moyens de prise de vue (26 ; 126) observant ledit reflet et engendrant des premiers signaux électriques (9) représentatifs de l'orientation de l'axe optique dudit oeil (5) ;
- des deuxièmes moyens de prise de vue (28 ; 128) observant ledit champ (6) et engendrant des deuxièmes signaux électriques (10) représentatifs dudit champ (6) et
- des moyens de calcul (8) recevant lesdits premiers et deuxièmes signaux électriques et délivrant des troisièmes signaux électriques (11) représentatifs de la portion (7i) dudit champ (6) regardée par ledit oeil (5) ;
caractérisé en ce qu'il comporte :
- un système optique de visée (2,3 ; 102) à travers lequel ledit oeil (5) regarde ledit champ (6), ledit système optique comprenant un oculaire de sortie (15 ; 117) derrière lequel est disposé ledit oeil (5) ; et
- un premier élément optique (22 ; 122) disposé du côté du plan focal objet dudit oculaire de sortie (15 ; 117), ledit premier élément optique laissant passer les rayons lumineux (16 ; 116) qui proviennent dudit champ (6) et qui traversent ledit système optique de visée et adressant audit oeil (5) ledit faisceau lumineux (24 ; 124) émis par ladite source de lumière (23 ; 123).

2. Dispositif selon la revendication 1,
caractérisé en ce que ledit premier élément optique (22 ; 122) est disposé dans le plan focal objet dudit oculaire de sortie (15 ; 117).

3. Dispositif selon l'une quelconque des revendications 1 ou 2,
caractérisé en ce que ledit premier élément optique (22), est, d'un côté, au moins partiellement transparent pour les rayons lumineux (16) provenant dudit champ (6) et, de l'autre côté, partiellement transparent, et partiellement réfléchissant pour ledit faisceau lumineux (24) émis par ladite source de lumière (23).

4. Dispositif selon la revendication 3,
caractérisé en ce qu'il comporte un second élément optique (21), couplé optiquement avec lesdits premiers et deuxièmes moyens de prise de vue (26,28) et disposé en amont (par rapport au sens de propagation des rayons lumineux (16) provenant dudit champ) dudit premier élément optique (22), de sorte que la partie (24') dudit faisceau lumineux (24) réfléchie par l'oeil et traversant ledit premier élément optique (22) est dirigée vers ledit second élément optique (21), ledit second élément optique (21) étant, d'un côté, partiellement transparent et partiellement réfléchissant pour les rayons lumineux (16) provenant dudit champ (6) et, de l'autre côté, au moins partiellement réfléchissant pour ledit faisceau lumineux (24) émis par ladite source de lumière.

5. Dispositif selon la revendication 4,
caractérisé en ce que ledit système optique de visée comporte un véhicule optique afocal (12 à 15) définissant un point de convergence intermédiaire (19), à l'emplacement duquel est disposé ledit second élément optique (21).

6. Dispositif selon la revendication 5,
caractérisé en ce que le système optique de visée comporte une lunette de visée (2), de laquelle est solidarisé ledit véhicule optique (12 à 15), celui-ci comportant un objectif (12) couplé optiquement à l'oculaire (17) de ladite lunette de visée (2) et un oculaire (15) formant ledit oculaire de sortie dudit système optique.

7. Dispositif selon l'une quelconque des revendications 1 ou 2,
caractérisé en ce que ledit premier élément optique (122), est, d'un côté, partiellement transparent et partiellement réfléchissant pour les rayons lumineux (116) provenant dudit champ (6) et, de l'autre côté, au moins partiellement réfléchissant pour ledit faisceau lumineux (124) émis par ladite source de lumière (123).

8. Dispositif selon la revendication 7,
caractérisé en ce que ledit premier élément optique (122) est couplé optiquement avec lesdits premiers et deuxièmes moyens de prise de vue (126,128), le couplage avec lesdits premiers moyens de prise de vue (126) étant obtenu grâce à un troisième élément optique (121), d'un côté au moins partiellement transparent et, de l'autre côté, au moins partiellement réfléchissant pour ledit faisceau lumineux (124) émis par ladite source de lumière (123) et permettant de séparer de ce faisceau (124) la partie (124') qui est réfléchie par l'oeil et par ledit premier élément optique (122).

9. Dispositif selon la revendication 8,
caractérisé en ce que lesdits premier et troisième éléments optiques (122,121) sont incorporés à une lunette de visée pour former ledit système optique (102), l'oculaire (117) de ladite lunette de visée formant l'oculaire de sortie dudit système optique.

10. Capteur (3) destiné à former avec une lunette de visée (2) un système optique de visée pour un dispositif destiné à déterminer la portion (7i) d'un champ (6) regardée par l'oeil (5) d'un observateur, ledit capteur comprenant :
- une source de lumière (23) adressant un faisceau lumineux (24) sur ledit oeil (5) pour former un reflet ;
- des premiers moyens de prise de vue (26) observant ledit reflet et engendrant des premiers signaux électriques représentatifs de l'orientation de l'axe optique dudit oeil (5) ;
- des deuxièmes moyens de prise de vue (28) observant ledit champ (6) et engendrant des deuxièmes signaux électriques représentatifs dudit champ ; et
- des moyens de calcul (8) recevant lesdits premiers et deuxièmes signaux électriques et délivrant des troisièmes signaux électriques représentatifs de la portion (7i) dudit champ (6) regardée par ledit oeil (5) ;
caractérisé en ce qu'il comporte :
- un véhicule optique afocal (12 à 15) solidarisé de ladite lunette de visée (2), ledit véhicule optique comportant un objectif (12) couplé à l'oculaire (17) de ladite lunette de visée et un oculaire (15) formant l'oculaire de sortie dudit système optique ;
- un premier élément optique (22) disposé du côté du plan focal objet dudit oculaire de sortie (15) et adressant audit oeil (5) ledit faisceau lumineux (24) émis par ladite source de lumière (23), ledit premier élément optique (22) étant, d'un côté, au moins partiellement transparent pour les rayons lumineux (16) provenant dudit champ et, de l'autre côté, partiellement transparent et partiellement réfléchissant pour ledit faisceau lumineux (24) émis par ladite source de lumière (23) ; et
- un second élément optique (21) couplé optiquement avec lesdits premiers et deuxièmes moyens de prise de vue (26,28) et disposé entre ledit objectif (12) et ledit premier élément optique (22), ledit second élément optique (21) étant, d'un côté, partiellement transparent et partiellement réfléchissant pour les rayons lumineux (16) provenant dudit champ et, de l'autre côté, au moins partiellement réfléchissant pour ledit faisceau lumineux (24) émis par ladite source de lumière (23).

11. Capteur selon la revendication 10,
caractérisé en ce que ledit premier élément optique (22) est disposé dans le plan focal objet dudit oculaire de sortie (15).

12. Capteur (3) selon l'une des revendications 10 ou 11,
caractérisé en ce qu'il incorpore ladite source de lumière (23).

13. Capteur (3) selon l'une des revendications 10 à 12,
caractérisé en ce que lesdits premiers et deuxièmes moyens de prise de vue (26,28) sont incorporés dans ledit capteur (3).

14. Système optique de visée pour un dispositif destiné à déterminer la portion (7i) d'un champ (6) regardée par l'oeil (5) d'un observateur, ledit système comprenant :
- une source de lumière (123) adressant un faisceau lumineux (124) sur ledit oeil (5) pour former un reflet ;
- des premiers moyens de prise de vue (126) observant ledit reflet et engendrant des premiers signaux électriques représentatifs de l'orientation de l'axe optique dudit oeil (5) ;
- des deuxièmes moyens de prise de vue (128) observant ledit champ (6) et engendrant des deuxièmes signaux électriques représentatifs dudit champ (6) ; et
- des moyens de calcul (8) recevant lesdits premiers et deuxièmes signaux électriques et délivrant des troisièmes signaux électriques, représentatifs de la portion (7i) dudit champ (6) regardée par ledit oeil (5) ;
ledit système optique étant caractérisé en ce qu'il comporte :
- une lunette de visée (102) pourvue d'un oculaire (117) ;
- un premier élément optique (122) disposé du côté du plan focal objet dudit oculaire (117) de la lunette de visée (102) et adressant audit oeil (5) ledit faisceau lumineux (124) émis par ladite source de lumière (123), ledit premier élément optique (122) étant, d'un côté, partiellement transparent et partiellement réfléchissant pour les rayons lumineux (116) provenant dudit champ (6) et, de l'autre côté, au moins partiellement réfléchissant pour ledit faisceau lumineux (124) émis par ladite source de lumière (123), ledit premier élément optique (122) étant optiquement couplé auxdits deuxièmes moyens de prise de vue (128) ; et
- un troisième élément optique (121), d'un côté au moins partiellement transparent et de l'autre côté partiellement réfléchissant pour ledit faisceau lumineux (124) émis par ladite source de lumière (123), ledit troisième élément optique (121) assurant la séparation entre ledit faisceau lumineux (124) et la partie (124') de celui-ci réfléchie par ledit oeil et ledit premier élément optique (122), ainsi que le couplage optique entre ledit premier élément optique (122) et lesdits premiers moyens de prise de vue (126).

15. Système optique selon la revendication 14,
caractérisé en ce que ledit premier élément optique (122) est disposé dans le plan focal objet dudit oculaire (117).

16. Système optique selon l'une des revendications 14 ou 15,
caractérisé en ce qu'il incorpore ladite source de lumière (123).

17. Système optique selon l'une des revendications 14 à 16,
caractérisé en ce que lesdits premiers et deuxièmes moyens de prise de vue (126,128) sont incorporés dans ledit système optique (102).

## Patentansprüche

1. Vorrichtung (1) zur Bestimmung des vom Auge (5) eines Beobachters beobachteten Abschnitts (7i) eines Feldes (6), mit:
- einer Lichtquelle (23; 123), aus der ein Lichtbündel (24; 124) an das Auge (5) gelangt und mindestens einen Reflex bildet;
- ersten Aufnahmemitteln (26; 126) zur Beobachtung des Reflexes und zur Erzeugung erster elektrischer Signale (9), die für die Richtung der optischen Achse von Auge (5) repräsentativ sind;
- zweiten Aufnahmemitteln (28; 128) zur Beobachtung von Feld (6) und zur Erzeugung zweiter elektrischer Signale (10), die für das Feld (6) repräsentativ sind, und
- Rechenmitteln (8), an die die ersten und zweiten elektrischen Signale gelangen und die dritte elektrische Signale (11) abgeben, die für den von Auge (5) beobachteten Abschnitt (7i) von Feld (6) repräsentativ sind,
dadurch gekennzeichnet, daß sie umfaßt:
- ein optisches Visiersystem (2,3; 102), durch das das Auge (5) das Feld (6) beobachtet, wobei das optische System ein Austrittsokular (15; 117) hat, hinter dem sich das Auge (5) befindet, und
- ein erstes optisches Element (22; 122) in der gegenstandsseitigen Brennebene des Austrittsokulars (15; 117), wobei das erste optische Element die durch das optische Visiersystem verlaufenden Lichtstrahlen (16; 116) von Feld (6) durchläßt und an das Auge (5) das Lichtbündel (24; 124) von der Lichtquelle (23; 123) gelangen läßt.

2. Vorrichtung nach Anspruch 1,
dadurch gekennzeichnet, daß das erste optische Element (22; 122) in der gegenstandsseitigen Brennebene des Austrittsokulars (15; 117) angeordnet ist.

3. Vorrichtung nach einem der Ansprüche 1 oder 2,
dadurch gekennzeichnet, daß das erste optische Element (22) einerseits die Lichtstrahlen (16) von Feld (6) zumindest teilweise durchläßt und andererseits das Lichtbündel (24) der Lichtquelle (23) teilweise durchläßt und teilweise reflektiert.

4. Vorrichtung nach Anspruch 3,
dadurch gekennzeichnet, daß sie ein zweites optisches Element (21) hat, das optisch mit den ersten und zweiten Aufnahmemitteln (26,28) gekoppelt und (bezogen auf die Fortpflanzungsrichtung der Lichtstrahlen (16) des Feldes) vor dem ersten optischen Element (22) angeordnet ist, so daß der vom Auge reflektierte und durch das erste optische Element (22) verlaufende Teil (24') des Lichtbündels (24) auf das zweite optische Element (21) gerichtet wird, wobei das zweite optische Element (21) einerseits die Lichtstrahlen (16) von Feld (6) teilweise durchläßt und teilweise reflektiert und andererseits das Lichtbündel (24) der Lichtquelle mindestens teilweise reflektiert.

5. Vorrichtung nach Anspruch 4,
dadurch gekennzeichnet, daß das optische Visiersystem ein afokales optisches Mittel (12 bis 15) hat, das einen Zwischen-Konvergenzpunkt (19) bildet, an dem das zweite optische Element (21) angeordnet ist.

6. Vorrichtung nach Anspuch 5,
dadurch gekennzeichnet, daß das optische Visiersystem ein Visierfernrohr (2) hat, mit dem das optische Mittel (12 bis 15) verbunden ist, wobei dieses ein Objektiv (12), das optisch mit dem Okular (17) des Visierfernrohrs (2) gekoppelt ist, und ein Okular (15) hat, das das Austrittsokular des optischen Systems darstellt.

7. Vorrichtung nach einem der Ansprüche 1 oder 2,
dadurch gekennzeichnet, daß das erste optische Element (122) einerseits die Lichtstrahlen (116) von Feld (6) teilweise durchläßt und teilweise reflektiert und andererseits das Lichtbündel (124) der Lichtquelle (123) zumindest teilweise reflektiert.

8. Vorrichtung nach Anspruch 7,
dadurch gekennzeichnet, daß das erste optische Element (122) optisch mit den ersten und zweiten Aufnahmemitteln (126,128) gekoppelt ist, wobei die Kopplung mit den ersten Aufnahmemitteln (126) durch ein drittes optisches Element (121) erreicht wird, das das Lichtbündel (124) der Lichtquelle (123) einerseits mindestens teilweise durchläßt und andererseits mindestens teilweise reflektiert und aus diesem Bündel (124) den Teil (124') heraustrennen kann, der vom Auge und vom ersten optischen Element (122) reflektiert wird.

9. Vorrichtung nach Anspruch 8,
dadurch gekennzeichnet, daß das erste und dritte optische Element (122,121) in ein Visierfernrohr eingebaut sind und das optische System (102) bilden, wobei das Okular (117) des Visierfernrohrs das Austrittsokular des optischen Systems bildet.

10. Sensor (3), der zusammen mit einem Visierfernrohr (2) ein optisches Visiersystem für eine Vorrichtung bildet, mit der der vom Auge (5) eines Beobachters beobachtete Abschnitt (7i) eines Feldes (6) bestimmt werden soll, wobei der Sensor umfaßt:
- eine Lichtquelle (23), von der aus ein Lichtbündel (24) an das Auge (5) gelangt und einen Reflex bildet;
- erste Aufnahmemittel (26) zur Beobachtung des Reflexes und zur Erzeugung der ersten elektrischen Signale, die für die Richtung der optischen Achse des Auges (5) repräsentativ sind;
- zweite Aufnahmemittel (28) zur Beobachtung von Feld (6) und zur Erzeugung der zweiten elektrischen Signale, die für das Feld repräsentativ sind, und
- Rechenmittel (8), an die die ersten und zweiten elektrischen Signale gelangen und die dritte elektrische Signale abgeben, die für den von Auge (5) beobachteten Abschnitt (7i) von Feld (6) repräsentativ sind;
dadurch gekennzeichnet, daß er umfaßt:
- ein afokales optisches Mittel (12 bis 15), das mit dem Visierfernrohr (2) verbunden ist, wobei das optische Mittel ein mit dem Okular (17) des Visierfernrohrs gekoppeltes Objektiv (12) und ein Okular (15) hat, das das Austrittsokular des optischen Systems bildet;
- ein erstes optisches Element (22), das in der gegenstandsseitigen Brennebene des Austrittsokulars (15) angeordnet ist und durch das das Lichtbündel (24) von Lichtquelle (23) an das Auge (5) gelangt, wobei das erste optische Element (22) einerseits die Lichtstrahlen (16) des Feldes mindestens teilweise durchläßt und andererseits das Lichtbündel (24) der Lichtquelle (23) teilweise durchläßt und teilweise reflektiert, und
- ein zweites optisches Element (21), das optisch mit den ersten und zweiten Aufnahmemitteln (26,28) gekoppelt und zwischen dem Objektiv (12) und dem ersten optischen Element (22) angeordnet ist, wobei das zweite optische Element (21) einerseits die Lichtstrahlen (16) des Feldes teilweise durchläßt und teilweise reflektiert und andererseits das Lichtbündel (24) der Lichtquelle (23) mindestens teilweise reflektiert.

11. Sensor nach Anspruch 10,
dadurch gekennzeichnet, daß das erste optische Element (22) in der gegenstandsseitigen Brennebene des Austrittsokulars (15) angeordnet ist.

12. Sensor (3) nach einem der Ansprüche 10 oder 11,
dadurch gekennzeichnet, daß in diesen die Lichtquelle (23) eingebaut ist.

13. Sensor (3) nach einem der Ansprüche 10 bis 12,
dadurch gekennzeichnet, daß die ersten und zweiten Aufnahmemittel (26,28) in den Sensor (3) eingebaut sind.

14. Optisches Visiersystem für eine Vorrichtung zur Bestimmung des vom Auge (5) eines Beobachters beobachteten Abschnitts (7i) eines Feldes (6), wobei das System umfaßt:
- eine Lichtquelle (123), von der aus ein Lichtbündel (124) an das Auge (5) gelangt und einen Reflex erzeugt;
- erste Aufnahmemittel (126) zur Beobachtung des Reflexes und zur Erzeugung der ersten elektrischen Signale, die für die Richtung der optischen Achse von Auge (5) repräsentativ sind;
- zweite Aufnahmemittel (128) zur Beobachtung des Feldes (6) und zur Erzeugung der zweiten elektrischen Signale, die für das Feld (6) repräsentativ sind, und
- Rechenmittel (8), an die die ersten und zweiten elektrischen Signale gelangen und die dritte elektrische Signale abgeben, die für den von Auge (5) beobachteten Abschnitt (7i) des Feldes (6) repräsentativ sind,
wobei das optische System dadurch gekennzeichnet ist, daß es umfaßt:
- ein Visierfernrohr (102) mit einem Okular (117);
- ein erstes optisches Element (122), das in der gegenstandsseitigen Brennebene des Okulars (117) des Visierfernrohrs (102) angeordnet ist und das Lichtbündel (124) der Lichtquelle (123) auf das Auge (5) richtet, wobei das erste optische Element (122) einerseits die Lichtstrahlen (116) von Feld (6) teilweise durchläßt und teilweise reflektiert und andererseits das Lichtbündel (124) von Lichtquelle (123) zumindest teilweise reflektiert, wobei das erste optische Element (122) optisch mit den zweiten Aufnahmemitteln (128) gekoppelt ist, und
- ein drittes optisches Element (121), das das Lichtbündel (124) der Lichtquelle (123) einerseits zumindest teilweise durchläßt und andererseits teilweise reflektiert, wobei das dritte optische Element (121) die Trennung zwischen dem Lichtbündel (124) und dem Teil (124') desselben, der vom Auge und vom ersten optischen Element (122) reflektiert wird, sowie die optische Kopplung zwischen dem ersten optischen Element (122) und den ersten Aufnahmemitteln (126) gewährleistet.

15. Optisches System nach Anspruch 14,
dadurch gekennzeichnet, daß das erste optische Element (122) in der gegenstandsseitigen Brennebene von Okular (117) angeordnet ist.

16. Optisches System nach einem der Ansprüche 14 oder 15,
dadurch gekennzeichnet, daß in diesem die Lichtquelle (123) eingebaut ist.

17. Optisches System nach einem der Ansprüche 14 bis 16,
dadurch gekennzeichnet, daß die ersten und zweiten Aufnahmemittel (126,128) in das optische System (102) eingebaut sind.

## Claims

1. Device (1) intended to determine the portion (7i) of a field (6) seen by the eye (5) of an observer and comprising:
- a light source (23; 123) sending a light beam (24; 124) on to the said eye (5) in order to form at least one reflection;
- a first image detector (26; 126) observing the said reflection and generating first electrical signals (9) representative of the orientation of the optic axis of the said eye (5);
- a second image detector (28; 128) observing the said field (6) and generating second electrical signals (10) representative of the said field (6); and
- computing means (8) receiving the said first and second electrical signals and delivering third electrical signals (11) representative of the portion (7i) of the said field (6) seen by the said eye (5);
characterized in that it includes:
- an optical sighting system (2, 3; 102) through which the said eye (5) looks at the said field (6), the said optical system comprising an exit eyepiece (15; 117) behind which the said eye (5) is placed; and
- a first optical component (22; 122) placed on the side of the object focal plane of the said exit eyepiece (15, 117), the said first optical component letting through the light rays (16, 116) which come from the said field (6) and which pass through the said optical sighting system and sending to the said eye (5) the said light beam (24; 124) emitted by the said light source (23; 123).

2. Device according to Claim 1, characterized in that the said first optical component (22; 122) is placed in the object focal plane of the said exit eyepiece (15; 117).

3. Device according to either one of Claims 1 and 2, characterized in that the said first optical component (22) is, on one side, at least partially transparent for the light rays (16) coming from the said field (6) and, on the other side, partially transparent and partially reflecting for the said light beam (24) emitted by the said light source (23).

4. Device according to Claim 3, characterized in that it includes a second optical component (21), coupled optically to the said first and second image detectors (26, 28) and placed upstream (with respect to the direction of propagation of the light rays (16) coming from the said field) of the said first optical component (22), so that the part (24') of the said light beam (24) reflected by the eye and passing through the said first optical component (22) is directed towards the said second optical component (21), the said second optical component (21) being, on one side, partially transparent and partially reflecting for the light rays (16) coming from the said field (6) and, on the other side, at least partially reflecting for the said light beam (24) emitted by the said light source.

5. Device according to Claim 4, characterized in that the said optical sighting system includes an afocal optical vehicle (12 to 15) defining an intermediate point of convergence (19), at the site of which the said second optical component (21) is placed.

6. Device according to Claim 5, characterized in that the optical sighting system includes a sighting telescope (2), to which the said optical vehicle (12 to 15) is firmly attached, the latter comprising an objective (12) coupled optically to the eyepiece (17) of the said sighting telescope (2) and an eyepiece (15) forming the said exit eyepiece of the said optical system.

7. Device according to either one of Claims 1 and 2, characterized in that the said first optical component (122) is, on one side, partially transparent and partially reflecting for the light rays (116) coming from the said field (6) and, on the other side, at least partially reflecting for the said light beam (124) emitted by the said light source (123).

8. Device according to Claim 7, characterized in that the said first optical component (122) is coupled optically to the said first and second image detectors (126, 128), the coupling with the said first image detector (126) being obtained by using a third optical component (121), on one side at least partially transparent and, on the other side, at least partially reflecting for the said light beam (124) emitted by the said light source (123) and making it possible to separate from this beam (124) the part (124') which is reflected by the eye and by the said first optical component (122).

9. Device according to Claim 8, characterized in that the said first and third optical components (122, 121) are incorporated in a sighting telescope in order to form the said optical system (102), the eyepiece (117) of the said sighting telescope forming the exit eyepiece of the said optical system.

10. Sensor (3) intended to form, with a sighting telescope (2), an optical sighting system for a device intended to determine the portion (7i) of a field (6) seen by the eye (5) of an observer, the said sensor comprising:
- a light source (23) sending a light beam (24) on to the said eye (5) in order to form a reflection;
- a first image detector (26) observing the said reflection and generating first electrical signals representative of the orientation of the optic axis of the said eye (5);
- a second image detector (28) observing the said field (6) and generating second electrical signals representative of the said field; and
- computing means (8) receiving the said first and second electrical signals and delivering third electrical signals representative of the portion (7i) of the said field (6) seen by the said eye (5);
characterized in that it includes:
- an afocal optical vehicle (12 to 15) attached firmly to the said sighting telescope (2), the said optical vehicle comprising an objective (12) coupled to the eyepiece (17) of the said sighting telescope and an eyepiece (15) forming the exit eyepiece of the said optical system;
- a first optical component (22) placed on the side of the object focal plane of the said exit eyepiece (15) and sending to the said eye (5) the said light beam (24) emitted by the said light source (23), the said first optical component (22) being, on one side, at least partially transparent for the light rays (16) coming from the said field and, on the other side, partially transparent and partially reflecting for the said light beam (24) emitted by the said light source (23); and
- a second optical component (21) coupled optically to the said first and second image detectors (26, 28) and placed between the said objective (12) and the said first optical component (22), the said second optical component (21) being, on one side, partially transparent and partially reflecting for the light rays (16) coming from the said field and, on the other side, at least partially reflecting for the said light beam (24) emitted by the said light source (23).

11. Sensor according to Claim 10, characterized in that the said first optical component (22) is placed in the object focal plane of the said exit eyepiece (15).

12. Sensor (3) according to one of Claims 10 and 11, characterized in that it incorporates the said light source (23).

13. Sensor (3) according to one of Claims 10 to 12, characterized in that the said first and second image detectors (26, 28) are incorporated in the said sensor (3).

14. Optical sighting system for a device intended to determine the portion (7i) of a field (6) seen by the eye (5) of an observer, the said system comprising:
- a light source (123) sending a light beam (124) on to the said eye (5) in order to form a reflection;
- a first image detector (126) observing the said reflection and generating first electrical signals representative of the orientation of the optic axis of the said eye (5);
- a second image detector (128) observing the said field (6) and generating second electrical signals representative of the said field (6); and
- computing means (8) receiving the said first and second electrical signals and delivering third electrical signals representative of the portion (7i) of the said field (6) seen by the said eye (5);
the said optical system being characterized in that it includes:
- a sighting telescope (102) provided with an eyepiece (117);
- a first optical component (122) placed on the side of the object focal plane of the said eyepiece (117) of the sighting telescope (102) and sending to the said eye (5) the said light beam (124) emitted by the said light source (123), the said first optical component (122) being, on one side, partially transparent and partially reflecting for the light rays (116) coming from the said field (6) and, on the other side, at least partially reflecting for the said light beam (124) emitted by the said light source (123), the said first optical component (122) being optically coupled to the said second image detector (128); and
- a third optical component (121), on one side at least partially transparent and on the other side partially reflecting for the said light beam (124) emitted by the said light source (123), the said third optical component (121) providing for the separation between the said light beam (124) and that part of it (124') reflected by the said eye and the said first optical component (122), and for the optical coupling between the said first optical component (122) and the said first image detector (126).

15. Optical system according to Claim 14, characterized in that the said first optical component (122) is placed in the object focal plane of the said eyepiece (117).

16. Optical system according to one of Claims 14 and 15, characterized in that it incorporates the said light source (123).

17. Optical system according to one of Claims 14 to 16, characterized in that the said first and second image detectors (126, 128) are incorporated in the said optical system (102).
